# EUROPÄISCHE PATENTSCHRIFT

(11) **EP 3 833 280 B1**
(45) Veröffentlichungstag und Bekanntmachung des Hinweises auf die Patenterteilung: **03.09.2025**
(21) Anmeldenummer: 19755823.2
(22) Anmeldetag: 29.07.2019
(51) Int. Cl.: A61B 17/50, A46B 9/02, A46B 9/12, A46B 13/02, A46B 13/08

(54) **ZECKENDREHER/TICK-ROTATOR**
DEVICE FOR GENTLE TICK REMOVAL BY ROTATION
DISPOSITIF D'ENLÈVEMENT DOUX DE TIQUES PAR ROTATION

(30) Priorität: 09.08.2018 EP 18000666; 13.04.2019 DE 102019002743
(43) Veröffentlichungstag der Anmeldung: 16.06.2021
(73) Patentinhaber: malipano GmbH, 95445 Bayreuth (DE); hecht international GmbH, 91595 Burgoberbach (DE)
(72) Erfinder: STEHR, Horst, 95447 Bayreuth (DE); STEHR, Isabell Kerstin, 95447 Bayreuth (DE)
(74) Vertreter: FDST Patentanwälte
(86) Internationale Anmeldenummer: PCT/EP2019/070351
(87) Internationale Veröffentlichungsnummer: WO 2020/030464

(56) Entgegenhaltungen:
- EP-A1- 1 384 418
- EP-A1- 3 607 903
- KR-A- 20050 002 114
- KR-A- 20090 110 151
- US-A- 4 865 482
- US-A1- 2013 324 802
- US-A1- 2015 065 927
- US-A1- 2015 182 415
- US-A1- 2017 049 076
- US-A1- 2018 098 615

## Beschreibung

Die Erfindung betrifft eine Vorrichtung zur sanften Entfernung von Zecken aus der Haut von Mensch und Tier.

Beim Entfernen eine kleine Zecke (Nymphe) mit einer Zeckenzange besteht die Gefahr, dass der Zeckenkörper abreißt, sodass der Stechapparat der Zecke ungreifbar in der Haut stecken bleibt. Zum Entfernen dessen verbleibt dann lediglich ein chirurgischer Eingriff oder das Herauswachsen-Lassen unter Beobachtung eines Arztes.

Zwar gibt es Erfindungen, aber diese sind nur bei ausgewachsenen Zecken ab etwa 2,5mm Körpergröße anwendbar und haben alle einen risikobehafteten Greif-, Schlingen- oder Hebelmechanismus. Diese sind allerdings nicht geeignet, um kleine Zecken (Nymphen) ohne die Gefahr des Abreißens des Zeckenkörpers aus der Haut zu entfernen.

Aus der EP 1 384 418 A1 ist eine Bürste zur Verwendung an einem zahnärztlichen Winkelstück bekannt, welche ein Borstenfeld aufweist, bei dem die Borsten, bezogen auf den Außendurchmesser des Borstenfeldes, eine von außen zur Mitte hin abnehmende Länge aufweisen.

In der US 2015/182415 A1 ist ein Gerät zur Augenlidpflege und ein Verfahren zur Verwendung eines solchen Geräts offenbart, wobei das Gerät hierzu einen oszillierenden Kopf aufweist.

In der US 2018/098615 A1 ist ein Keratin-Behandlungskit beschrieben, anhand dessen es eine halbfeste oder feste, nicht-newtonsche kosmetische Zusammensetzung auf eine Oberfläche von keratinischem Gewebe auftragbar ist. Dieses umfasst einen Hybridapplikator, welcher motorisch oszillierend antreibbar ist.

Aus der US 2013/324802 A1 ist ein handgehaltenes Werkzeug zum Auffinden und Entfernen von parasitären Insekten auf haarbedeckter Haut bekannt.

Aus der KR 2005 0002114 A ist eine multifunktionale Bürste bekannt, die zum Trimmen von Haaren von Haustieren unter Verwendung der von einem Staubsauger erzeugten Absorptionskraft und zur Sterilisation von Viren durch Ausstrahlung von ultravioletten Strahlen und Ultraschallwellen dient.

**In** der US 2017/049076 A1 ist ein Tierreinigungswerkzeug mit einem oberen Gehäuseabschnitt und mit einer Bodenplattenoberfläche dargestellt, wobei die Bodenplattenoberfläche einen Satz von Bürstenelementen aufweist, die voneinander beabstandet sind und von der Bodenplatte nach außen vorstehen.

Aus der US 4 865 482 A ist eine Floh- und Zeckenbürste für Haustiere bekannt, deren Bürstenkopf eine Vielzahl von kegelstumpfförmigen hohlen Zähnen mit einer Auslassöffnung aufweist, durch die Inhaltsstoffe aus einem mit dem Bürstenkopf verbundenen Behälter austreten können.

Der Erfindung liegt die Aufgabe zugrunde, eine Vorrichtung anzugeben, anhand welcher eine bereits in die Haut eines Wirts gestochene Zecke durch Rotation entfernt werden kann, wobei eine Verletzung der Zecke vermieden werden soll.

Hinsichtlich der Vorrichtung wird diese Aufgabe durch die Merkmale des Anspruchs 1 gelöst. Bevorzugte Ausführungsformen sind Gegenstand der Unteransprüche.

Erfindungsgemäß ist also eine auch als Zeckendreher oder als Tick-Rotator bezeichnete Vorrichtung zur Entfernung von Zecken aus der Haut eines Menschen oder eines Tieres vorgesehen. Die Vorrichtung weist auf: ein Gehäuse mit einem an diesem ausgebildeten Griff; einen drehbaren Pinselaufsatz; und eine Aufnahmevorrichtung zur Aufnahme des Pinselaufsatzes; wobei der Pinselaufsatz eine Haaranordnung mit Pinselhaaren aufweist, die aus einer Gruppe ausgewählt sind, welche Gruppe Kunsthaare und Naturhaare aufweist, und wobei die Haaranordnung des Pinselaufsatzes eine mittig vorgesehene Vertiefung für das Platzieren eines Zeckenkörpers ausbildet.

Erfindungsgemäß ist eine durchsichtige Zentrierhülse aus Kunststoff oder Glas abnehmbar am Gehäuse aufgesteckt, wobei die Zentrierhülse mit dem freien Ende der Randpinselhaare abschließt.

Der Erfindung liegen folgende Überlegungen zugrunde:
Beim Entfernen der Zecke lediglich durch den vorsichtigen Zug der Zecke nach oben, also den Widerhaken der Zecke entgegengesetzt, mit einem Greifwerkzeug (z.B. Pinzette, Schlinge, Karte oder Zange) ist es ein riskantes "Kräftezugspiel" zwischen Zecke und dem Entferner. Auch das Verfahren einer Zeckenschlinge basiert auf dem Ergreifen der Zecke und einem anschließenden riskanten Zug. Ein Entfernen ist somit immer risikobehaftet. Greift ein Werkzeug minimalst (1/10 Millimeter) zu hoch, wird die Zecke gequetscht oder/ und abgerissen. Greift es zu tief, wird der Patient schmerzhaft gezwickt und die bereits gereizte, gestochene Hautoberfläche wird zusätzlich unnötig belastet. Bei den kleinen Larven und Nymphen, noch dazu an schwer zugänglichen Körperstellen, ist ein sicheres Entfernen bzw. ein sicheres Greifen vergleichsweise schwierig. Larven sind selbst mit bloßem Auge kaum erkennbar. Hier ist eine Rotation mit Greifmechanismen quasi unmöglich.

Ab der Größe einer ausgewachsenen Zecke schrumpft zwar das Risiko des Abreißens der Zecke mit einem Greifwerkzeug gegenüber der Nymphe, bleibt aber auch hier dennoch bestehen. Alle bisher bekannten Vorrichtungen müssen die Zecke zuerst greifen und dann entfernen. Keine dieser Vorrichtungen kann eine Larve der Größe von 0,1mm erfassen.

Weiterhin ist festzuhalten, dass eine Zecke kein Gewinde hat oder sich nicht kreisförmig, wie eine Schraube, in die Haut des Wirtes bohrt. Ferner sticht eine Zecke in die Haut und "sägt" ihre beiden parallel angeordneten "Stechgraber" (Cheliceren) abwechselnd geradlinig in die Haut. Die seitlich daran angeordneten winzigen Widerhaken sorgen u.a. für den festen Halt der Zecke im Wirt. Dennoch muss die Zecke diesen Mechanismus bzw. ihre Verankerung in der Haut lösen können, da sie bis zu ihrer geschlechtlichen Ausreifung zur "erwachsenen" Zecke vorher zwei Entwicklungsstadien durchlaufen muss. Sie wird als Larve geboren, wird dann zur Nymphe und schließlich zur männlichen oder weiblichen Zecke. Zwischen jeder dieser Stadien häutet sie sich und braucht jedes Mal unbedingt wieder eine neue Einstichstelle.

Folglich muss eine Zecke also bis zu ihrer Ausreifung dreimal ihren Wirt bzw. ihre Eistichstelle wechseln und somit bis zum Ende auch dreimalloslassen können. Ungeklärt ist, ob sich eine Zecke während der Zeit in der Haut des Wirtes dreht oder sie ihren Stechapparat beim Verlassen des Wirtes drehend aus der Haut zieht.

Man muss die Zecke also nur zum Loslassen bringen bzw. stimulieren.

Je öfter man die Zecke behutsam um ihre Achse dreht, umso leichter lässt sie sich entfernen. Nach fünf Umdrehungen ist Grenze spätestens erreicht. Ein Problem dabei ist nur, wie man eine winzige Zeckenlarve ab 0,1mm drehen soll, ohne sie zu greifen oder zu quetschen. Für größere Zecken gibt es genügend Vorrichtungen und das Risiko des Abreißens wird immer kleiner, je größer die Zecke wird, besteht allerdings grundsätzlich weiter.

Wenn man die Zecke behutsam, gleichmäßig und dauerhaft dreht indem man auch den Greifreflex der Beine nutzt, verhängen diese sich im speziell zugeschnittenen Zeckenpinsel und die Zecke wird mehrfach, unaufhaltsam, komplett, sanft und langsam gedreht.

Nach etwa fünf bis sechs Umdrehungen (entspricht etwa1 bis 4 Rotationsintervallen von ca. 10 Sekunden Dauer) des Zeckenkörpers wird es für die Zecke "untragbar", sie lässt los und sucht sich unversehrt eine neue Einstichstelle. So, wie sie es aus der Natur kennt.

Vorteilhaft funktioniert dies auch bei kleinen Larven und Nymphen, die den Menschen hauptsächlich stechen. Der speziell kegelförmig ausgehöhlte Pinsel umschließt zudem das Tier ohne Gefahr komplett und dreht dieses bis es loslässt. Nur ein kitzelndes Gefühl macht sich auf der Haut des Gestochenen Wirtes (Mensch oder Tier) bemerkbar.

Im Vergleich zu Zeckenrotationen mit der Hand oder herkömmlichen Greifwerkzeugen, Schlingen, Karten und Pinzetten, besteht hier kein Abriss-Risiko.

Der Tick-Rotator tut nicht weh und zwickt nicht. Er ist kinderleicht zu bedienen, transportabel und somit gerade auch bei kleinen Kindern und schwer zugänglichen Körperstellen sehr gut anwendbar.

Alle herkömmlichen Vorrichtungen zum Entfernen einer Zecke mit oder ohne Rotation basieren auf einem Greif-, Schlingen- oder Hebelmechanismus an Zecken. Bei allen Geräten, auch mit einer Rotation muss die Zecke vorher in irgendeiner Form gegriffen werden um sie dann danach drehen zu können.

Der Tick-Rotator (Zecken-Dreher) basiert auf der sanften Entfernung einer bereits zugestochenen Zecke aus der Haut ihres Wirtes. Hierbei wird die Zecke nicht mit einem Greifmechanismus wie Pinzette, Schlinge, Karte oder Zange gegriffen, sondern in einen, speziell für Zecken zugeschnittenen Pinsel eingebettet, durch eine sanfte kreisende Rotation drehend stimuliert und zum Loslassen bewegt.

Die Zecke wird durch elektrische, gleichmäßige und langsame Pinselrotationsintervalle in eine für sie "unbequeme" Situation gebracht, sodass sie ihren verankerten Stechapparat löst um nach einer anderen "angenehmeren" Einstichsteile am Wirt zu suchen.

Somit kommt es für die Zecke bei der Entfernung mit dem Tick-Rotator zu keiner Panik-, Todesangst- oder Stresssituation wie z.B. durch Quetschung, Greifen oder Körperteilabtrennung mit einer Pinzette oder Zange und einer damit eventuell verbundenen Gefahr der bakteriellen Übertragung (z.B. Borreliose).

Gerade kleinere Zecken, also Larven oder Nymphen haben vor ihrer Häutung so winzig filigrane Stechwerkzeuge, dass ein perfektes Greifen nahezu unmöglich ist oder weil sich der Patient, zum Beispiel ein Kind, nicht lange genug ruhig verhält. Hier ist leider auch die feinste medizinische Spezialpinzette noch zu groß.

Genau hierfür gibt es den Tick-Rotator. Er ist extra speziell genau so konstruiert und vorne am Pinsel so geschnitten, dass er auch die kleinste Larve komplett entfernt.

Vorzugsweise ist ein elektrischer Antrieb für das Drehen des Pinselaufsatzes vorgesehen.

Bevorzugst ist ferner einem Elektromotor vorgesehen, der eine Antriebswelle aufweist, und mit einem Getriebe, wobei der Elektromotor gestaltet ist, um das Getriebe mit seiner Antriebswelle anzutreiben.

Es kann vorgesehen sein, dass der Elektromotor und das Getriebe so gestaltet sind, dass ausgangsseitig des Getriebes eine Drehzahl von 60 bis 150 Umdrehungen pro Minute erzeugbar ist.

Ferner kann vorgesehen sein, dass der Elektromotor und das Getriebe in dem Gehäuse angeordnet sind.

In bevorzugter Gestaltung ist die Aufnahmevorrichtung außerhalb des Gehäuses angeordnet ist.

In bevorzugter Gestaltung ist die Aufnahmevorrichtung zur Aufnahme des Pinselaufsatzes eine Aufnahmevorrichtung zur lösbaren Aufnahme des Pinselaufsatzes.

In vorteilhafter Ausgestaltung ist ein Gehäusedeckel vorgesehen, der als Schraubdeckel gestaltet ist und auf das Gehäuse schraubbar ist.

Der Gehäusedeckel kann beispielsweise mit einem Ein- und Ausschalter versehen sein.

In vorteilhafter Ausgestaltung kann eine Batterie oder ein Akku als Energieträger vorgesehen und in dem Gehäuse angeordnet sein.

Vorzugsweise weist der Pinselaufsatz für seine Fixierung in der Aufnahmevorrichtung rückseitig einen Aufnahmestift aus Metall oder Kunststoff auf.

Vorzugsweise weist der Pinselaufsatz eine Haaranordnung mit Pinselhaaren aufweist, die aus einer Gruppe ausgewählt sind, welche Gruppe Kunsthaare und Naturhaare auf, wobei die Haaranordnung des Pinselaufsatzes eine mittig vorgesehene Vertiefung für das Platzieren eines Zeckenkörpers ausbildet.

Zum Verändern der Stellung des Verankerungsmechnismus einer Zecke aus einer Verankerungsstellung in eine gelöste Stellung, wobei die Zecke bei dieser Stellungsveränderung derart unversehrt bleibt, dass sie nach der Stellungsveränderung weiterhin in der Lage ist zu krabbeln, wird der Pinselaufsatz der Vorrichtung, welcher Pinselaufsatz eine Haaranordnung mit Pinselhaaren aufweist, die mittig die Vertiefung ausbildet, mit seiner Vertiefung auf die Zecke aufgesetzt und anschließend gedreht, so dass die Zecke aus Ihrer Verankerungsstellung in eine gelöste Stellung übergeht. Insbesondere kann dies erfolgen, in dem die Zecke mittels des Pinselaufsatzes gedreht wird. Vorzugsweise ist die Vertiefung im Bereich der Maß- und oder Formangaben, die in Bezug auf bespielhafte erfindungsgemäße Pinselaufsätze in dieser Anmeldung angegeben sind. Dies ermöglicht in bevorzugter Gestaltung, dass die Beine der Zecke von der rotierenden Haaranordnung mitgenommen werden und so die Zecke gedreht wird.

Zum Lösen einer in einer menschlichen oder tierischen Haut verankerten Zecke aus der Verankerungsstellung, wobei die Zecke, die die Fähigkeit hat zu krabbeln, bei diesem Lösen derart unversehrt bleibt, dass sie nach dem Lösen krabbeln kann, wird ferner der Pinselaufsatz, der die Haaranordnung mit Pinselhaaren aufweist, die mittig eine Vertiefung ausbildet, mit seiner Vertiefung auf die Zecke aufgesetzt und anschließend gedreht wird, so dass die Zecke aus Ihrer Verankerung in der Haut gelöst wird. Dabei ist insbesondere vorgesehen, dass der Pinselaufsatz so positioniert wird, dass die Zecke in der Vertiefung des der Haaranordnung angeordnet ist.

Das Drehen der Zecke kann beispielsweise mittels eines elektrischen Motors erfolgen, dessen Motorwelle mit einem Pinselaufsatz zur Drehung des Pinselaufsatzes gekoppelt ist.

In besonders zu bevorzugender Ausgestaltung wird vor dem Aufsetzen des Pinselaufsatzes auf die Zecke eine Flüssigkeit, vorzugsweise Wasser, auf den Bereich aufgebracht, in dem sich die Zecke befindet. Dies ist insbesondere zweckmäßig bei Tieren mit einem Unterfeil bzw. bei Menschen und Tieren, bei denen sich die Zecke mit ihrer Einstichsteile an einer stark behaarten Körperstelle befindet. Z. B. kann diese Maßnahme bei Menschen im Kopfbereich oder auch bei einer Katze oder einem Hasen ergriffen werden. Hierdurch lässt sich die Zecke bei starker Behaarung am Menschen oder bei Tieren wie z.B. Katzen, Hasen bzw. Tieren mit Fell und Unterteil/ Flaum bzw. bei Katzen bzw. Tieren mit Unterfeil leichter über die Rotation des Pinselaufsatzes entfernen.

In besonders zu bevorzugender Ausgestaltung erfolgt das Drehen des Pinselaufsatzes gleichmäßig und in mehreren Intervallen.

Ferner ist ein Pinselaufsatz für eine erfindungsgemäße Vorrichtung vorgesehen, wobei der Pinselaufsatz eine Haaranordnung mit Pinselhaaren aufweist, die aus einer Gruppe ausgewählt sind, welche Gruppe Kunsthaare und Naturhaare aufweist, und wobei die Haaranordnung des Pinselaufsatzes eine mittig vorgesehene Vertiefung für das Platzieren eines Zeckenkörpers ausbildet.

Im Folgenden sollen einige bevorzugte Gestaltungendes Pinselaufsatzes erläutert werden, wobei sich diese Gestaltungen auf Weiterbildungen des Pinselaufsatzes als solchem, sowie auf Weiterbildungen des Pinselaufsatzes der erfindungsgemäßen Vorrichtung beziehen: Die Vertiefung der Haaranordnung des Pinselaufsatzes kann ist insbesondere so, dass sie sich von den freien Enden der Haaranordnung in das Innere der Haaranordnung erstreckt. Bevorzugt nimmt in Tiefenrichtung der Vertiefung die Querschnittsfläche zu, beispielsweise monoton oder streng monoton zu.

In bevorzugter Gestaltung ist die Vertiefung, die in der Haaranordnung des Pinselaufsatzes vorgesehen ist, als Kegel oder Paraboloid ausgebildet. Die Vertiefung der Haaranordnung des Pinselaufsatzes kann aber auch beispielsweise zylindrisch sein.

In bevorzugter Gestaltung ist die Haaranordnung des Pinselaufsatzes außen zylindrisch geformt. Aber auch eine konische Ausgestaltung, wie z.B. ein zu den freien Enden der Haare konisch auseinanderlaufende äußere Kontur der Haaranordnung des Pinselaufsatzes ist bevorzugt.

Der Pinseldurchmesser ist vorzugsweise außen größer als 3 mm, bevorzugt, größer als 3 mm und kleiner als 40 mm, bevorzugt größer als 3 mm und kleiner als 30 mm, größer als 3 mm und kleiner als 20 mm, bevorzugt größer als 4 mm und kleiner als 15 mm, bevorzugt größer als 4 mm und kleiner als 10 mm, z.B. 5 mm oder 6 mm oder 7 mm oder 8 mm.

Die Haarlänge der radial äußeren positionierten Haare der Haaranordnung des Pinselaufsatzes ist vorzugsweise größer oder gleich 1 mm, bevorzugt größer oder gleich 2 mm, bevorzugt größer oder gleich 3 mm, bevorzugt größer oder gleich 4 mm.

Besonders bevorzugt ist die Haarlänge der radial äußeren positionierten Haare der Haaranordnung des Pinselaufsatzes kleiner als 10 mm. **In** besonders zu bevorzugender Gestaltung ist die Haarlänge der radial äußeren positionierten Haare der Haaranordnung des Pinselaufsatzes größer oder gleich 1 mm und kleiner oder gleich 5 mm, bevorzugt größer oder gleich 2 mm und kleiner oder gleich 4 mm. Es kann, insbesondere dabei, vorgesehen sein, dass die die Haarlänge der radial äußeren positionierten Haare der Haaranordnung des Pinselaufsatzes jeweils konstant ist.

Die Tiefe der Vertiefung der Haaranordnung des Pinselaufsatzes kann beispielsweise größer oder gleich 1mm und kleiner oder gleich 10 mm, z.B. größer oder gleich 1 mm und kleiner oder gleich 8 mm, z.B. größer oder gleich 1 mm und kleiner oder gleich 6 mm, z.B. größer oder gleich 1 mm und kleiner oder gleich 4 mm, z.B. größer oder gleich 2 mm und kleiner oder gleich 3 mm sein.

Die Vertiefung der Haaranordnung des Pinselaufsatzes kann beispielsweise runde Querschnitte senkrecht zu ihrer Tiefenrichtung aufweisen. Beispielsweise kann dabei der Durchmesser dieser Querschnitte größer oder gleich 0 mm und kleiner oder gleich 6 mm, z.B. größer oder gleich 0,1 mm und kleiner oder gleich 4 mm, oder größer oder gleich 0,1 mm und kleiner oder gleich 1 mm, oder größer oder gleich 0,1 mm und kleiner oder gleich 0,8 mm, oder größer oder gleich 0 mm und kleiner oder gleich 2 mm sein. Dabei kann insbesondere vorgesehen sein, dass der genannte Durchmesser innerhalb der Öffnung konstant ist. Es kann aber auch vorgesehen sein, dass der genannte Durchmesser innerhalb der genannten Grenzen liegt und variiert. Zum Beispiel kann die jeweils genannte Untergrenze dem Durchmesser des kleinsten Querschnitts einer betreffenden Vertiefung der Haaranordnung des Pinselaufsatzes entsprechen, und die jeweils genannte Obergrenze dem Durchmesser des größten Querschnitts der gleichen betreffenden Vertiefung der Haaranordnung des Pinselaufsatzes entsprechen. Insbesondere kann als die genannte Vertiefung ein Paraboloid sein, dessen minimaler Durchmesser 0,0 mm beträgt und dessen maximaler Durchmesser 1 mm beträgt, oder ein Paraboloid sein, dessen minimaler Durchmesser 0,1 mm beträgt und dessen maximaler Durchmesser 0,8 mm beträgt, ein Paraboloid sein, dessen minimaler Durchmesser 0,8 mm beträgt und dessen maximaler Durchmesser 2 mm beträgt, ein Paraboloid sein, dessen minimaler Durchmesser 0,0 mm beträgt und dessen maximaler Durchmesser 1 mm beträgt, ein Paraboloid sein, dessen minimaler Durchmesser 0,0 mm beträgt und dessen maximaler Durchmesser 4 mm beträgt, oder ein Paraboloid sein, dessen minimaler Durchmesser 2 mm beträgt und dessen maximaler Durchmesser 3 mm beträgt. Besonders bevorzugt ist die genannte Vertiefung ein Paraboloid sein, dessen minimaler Durchmesser größer oder gleich 0,0 mm und kleiner oder gleich 2 mm beträgt und dessen maximaler Durchmesser größer oder gleich 0,8 mm und kleiner oder gleich 5mm beträgt, wobei der maximale Durchmesser dabei größer als der minimale Durchmesser ist.

Es kann vorgesehen sein, dass das Drehen des Pinselaufsatzes jeweils im Uhrzeigersinn erfolgt. **In** alternativer Ausgestaltung kann das Drehen des Pinselaufsatzes jeweils im Gegenuhrzeigersinn erfolgen.

Vor Gebrauch, insbesondere eine bevorzugten Ausführungsform der Erfindung, wird der Energieträger/ Batterie in den Tick-Rotator eingesetzt. Danach wird vorne die Zentrierhülse abgenommen, um den gewählten Pinselaufsatz in die Aufnahmevorrichtung zu stecken. Daraufhin wird die Zentrierhülse wieder montiert.

Durch das leichte Aufsetzen des Pinsels und der Hülse mit dem Zeckenkörper in der dafür vorgesehenen Vertiefung in der Mitte des Pinsels verhängt sich die bereits eingestochene Zecke mit ihren Beinen und ihrem Körper zwangsläufig in den einzelnen Pinselhärchen.

Wird nun die erste Rotation durch Drücken des Schalters am Tick-Rotator nach links oder rechts gestartet, dreht sich die Zecke mit. Jedoch dreht sich die Zecke nicht bei jeder Pinselumdrehung mit. Deshalb braucht man bei Bedarf bis zu vier Rotationsintervalle.

Je nach Größe der Zecke und ihrem angeborenen Greifreflex löst sie sich nach spätestens fünf Umdrehungen um ihre eigene Achse aus der Einstichsteile und sucht sich unversehrt und lebendig eine neue.

Bei einer Larve (0,1mm bis 0,8mm) oder Nymphe (0,8mm bis 2,0mm) kann es aufgrund ihrer geringen Körpergröße zu mehreren Intervallen sanfter gleichmäßiger Rotation des Pinsels bedürfen bis sie schließlich loslässt, da die winzigen Zeckenbeine und der Körper schwerer zu drehen sind.

Hierbei ist es wichtig, dass jedes Rotationsintervall mit Zirkulation des Pinsels immer kontinuierlich und ohne Ruckeln und Druck stattfindet. Es darf kein Druck auf die Zecke bzw. auf die Haut ausgeübt werden. Borsten könnten den winzigen Zeckenkörper verletzen oder Quetschen. Ein leichtes Aufsetzen der weichen Pinselhaare auf die Haut des Wirtes bzw. der Zecke reicht aus. Hierzu dient die durchsichtige Zentrierhülse als Unterstützung.

Jede Anwendung wird in Intervallen durchgeführt. Jedes Benutzerintervall, auch das Startintervall zu Beginn dauert ca.10 Sekunden. Je nach Zeckengröße und Körpereinstichstelle kann der Benutzer die Intervalle beliebig wiederholen. Zwischen den Intervallen nutzt der Anwender die Unterbrechung für eine vorsichtige Sichtung und Überprüfung der Zecke durch anheben der Zentrierhülse und des Pinsels, ob sie schon losgelassen hat oder nicht. Sollte die Zecke noch festsitzen, wird das nächste 10-Sekundeintervall durchgeführt. Bei Larven hat sich manchmal gezeigt, dass bis zu vier Intervalle notwendig sind. Für größere Zecken, die sich bereits auf einen Körperdurchmesser von ca. 5mm und mehr vollgesaugt haben ist der Tick-Rotator nicht zu empfehlen, da die Zecke dann so groß geworden ist, dass ihr Körper mit dem Pinsel nicht mehr im Sinne der Beschreibung erfasst werden kann.

Die Intervalle mit ihren sanften langsamen Rotationen und den kurzen Pausen assoziieren der Zecke eine natürliche, nicht belastende Beeinflussung, die sie auch aus der Natur durch kratzen ihres Wirtes oder durch am Wirt streifende Äste oder Rinde kennt.

Nachfolgend werden Ausführungsbeispiele der Erfindung anhand einer Zeichnung näher erläutert. Darin zeigen:
- Fig. 1: schematisch eine Vorrichtung zum Entfernen von Zecken aus der Haut eines Wirts, wobei die Vorrichtung einen Pinselaufsatz aufweist,
- Fig. 2: schematisch den Pinselaufsatz in Seitenansicht
- Fig. 3: schematisch den Pinselaufsatz in Draufsicht auf dessen Haaranordnung, und
- Fig. 4: eine in die Haut eines Wirtes eingestochene Zecke, wobei der Zeckenkörper in einer parabelförmigen Vertiefung des Pinselaufsatzes angeordnet ist.

Einander entsprechende Teile und Größen sind in allen Figuren stets mit gleichen Bezugszeichen versehen.

Die auch als Tick-Rotator oder Zeckendreher bezeichnete Vorrichtung 1 hat eine handliche, zylindrische Stiftform ähnlich wie eine kleine Stabtaschenlampe mit einer beidseitigen Vertiefung im Gehäuse, um ihn ähnlich wie einen Füllfederhalter griffsicher und präzise bedienen zu können. Dessen Länge beträgt insgesamt ca.12 bis 15cm.

Der Durchmesser des Gehäuses beträgt ca.1,5 bis 3cm.

Der Tick-Rotator wird hauptsächlich elektrisch betrieben, um für jeden Benutzer immer dasselbe, gleichmäßige, langsame und dauerhafte Rotationsintervall gewährleisten zu können. Eine ausgefeilte rein mechanische Version wäre denkbar. Dabei ist aber nur schwer eine durch den Laien immer wieder gleiche Benutzung realisierbar.

Diesbezüglich setzt sich der elektrische Tick-Rotator aus sechs nacheinander zusammengebauten Bauelementen zusammen:
1. Pinselaufsätze
2. Aufnahmevorrichtung für Pinselaufsätze
3. Elektromotor mit Getriebe und Antriebswelle
4. Gehäuse mit Batterie und Verkabelung
5. Gehäusedeckel mit Schalter
6. Zentrierhülse

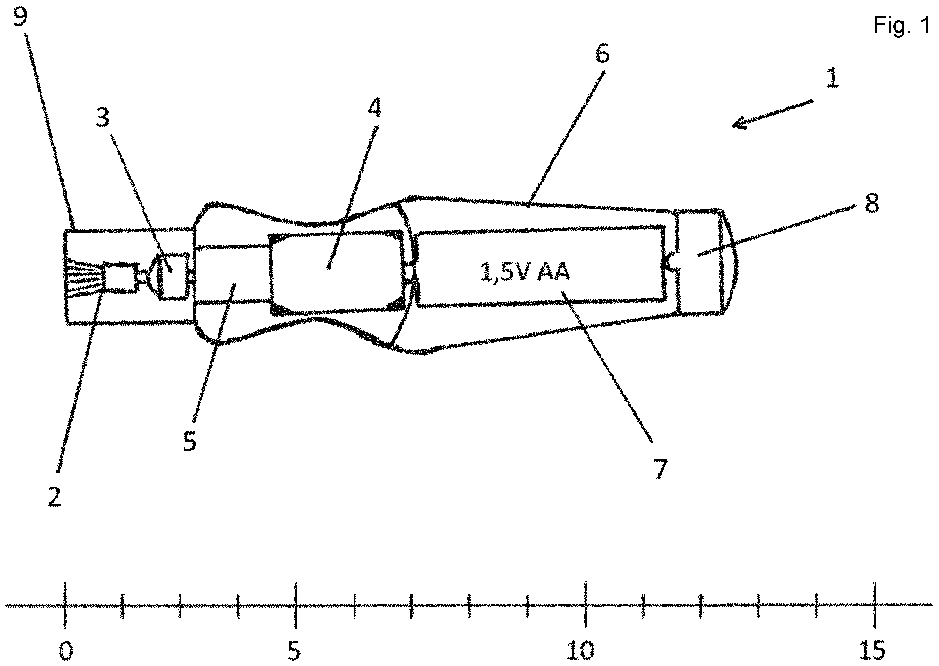

In der Fig. 1 ist die Vorrichtung mit dem Bezugszeichen 1, der Pinselaufsatz mit dem Bezugszeichen 2, die Aufnahmevorrichtung für den Pinselaufsatz mit dem Bezugszeichen 3, ein Elektromotor mit dem Bezugszeichen 4, ein Getriebe mit mehr als 300 U/min mit dem Bezugszeichen 5, das Gehäuse mit Griff mit dem Bezugszeichen 6, eine Batterie oder ein Akku als Energieträger mit dem Bezugszeichen 7, der Gehäusedeckel mit Schalter mit dem Bezugszeichen 8 und die Zentrierhülse mit dem Bezugszeichen 9 versehen.

### 1. Pinselaufsätze:

Es gibt z.B. drei Aufsatzgrößen mit jeweils verschiedengroß geschnittenen Vertiefungen und Zuschnitten. Larven, Nymphen und Zecken. Für jedes Entwicklungsstadium der Zecke einen passenden Aufsatz.

### 1.1. Aufsatzpinsel (Pinselaufsatz) "klein für Larven" (ca. 0.1mm bis 0,8mm):

- Pinseldurchmesser: ca.5mm
- Pinselhaarlänge Randhaare: größer oder gleich 2 mm, z.B. 3 mm oder 4 mm oder 5 mm oder ca.6mm
- Kegeldurchmesser: ca.O,1 bis 0,8mm
- Kegelhöhe: ca.1,5mm

### Gemäß eines weiteren Beispiels:

- Pinselkranzdurchmesser außen: ab 2mm
- Durchmesser der Querschnitte der Vertiefung von oben ungleichmäßig parabelförmig abnehmend: Beginnend mit 1mm bis 0 mm
- Höhe der Vertiefung: bis 2mm

### 1.2. Aufsatzpinsel (Pinselaufsatz) "mittel für Nymphen" (ca. 0,8mm bis 2mm):

- Pinseldurchmesser: ca.5mm
- Pinselhaarlänge Randhaare: größer oder gleich 2 mm, z.B. 3 mm oder 4 mm oder 5 mm oder ca.6mm.
- Kegeldurchmesser: ca.0,8 bis 2mm Kegelhöhe: ca. 2,5mm

### Gemäß eines weiteren Beispiels:

- Pinselkranzdurchmesser außen: ab 3mm
- Durchmesser der Querschnitte der Vertiefung von oben ungleichmäßig parabelförmig abnehmend: Beginnend mit 2mm bis 0 m
- Höhe der Vertiefung: 2 bis 3mm

### 1.3. Aufsatzpinsel (Pinselaufsatz) "groß für geschlechtsreife Zecken" (ca. 2mm bis 3,5mm):

- Pinseldurchmesser: ca.7mm
- Pinselhaarlänge Randhaare: größer oder gleich 4 mm, z.B. 4 mm oder 5 mm oder ca.6mm
- Kegeldurchmesser: ca.2mm bis 3,0mm
- Kegelhöhe: ca. 3,5mm

### Gemäß eines weiteren Beispiels:

- Pinselkranzdurchmesser außen: ab 5mm
- Durchmesser der Querschnitte der Vertiefung von oben ungleichmäßig parabelförmig abnehmend: Beginnend mit 4mm bis 0 mm.
- Höhe der Vertiefung: 3 bis 4mm

Jeder Pinselaufsatz 2 hat eine runde, dichte Haaranordnung 11 (Rundpinsel). Die Pinselhaare bestehen aus weichen Kunst- oder Naturhaaren wie z.B. Ziegenhaare. Es werden keine Borsten verwendet, da die Zecke nicht unnötig belastet oder gereizt werden darf. Der Pinsel hat je nach Größe eine dementsprechend nach innen kegelförmig geschnittene, kleine Vertiefung 12 an der geraden Pinselauflagefläche.

Diese Kegelvertiefung 12 dient dazu, sie über den Zeckenkörper 14 zu platzieren ohne diesen zu quetschen, sodass sich die Zecke 14 mit dem Pinsel dreht.

Der Pinsel hat an seiner Rückseite einen Aufnahmestift 10 aus Metall oder Kunststoff um ihn in der Aufnahmevorrichtung 3 zu fixieren.

### Gemäß eines weiteren Beispiels und einer weiteren Beschreibung:

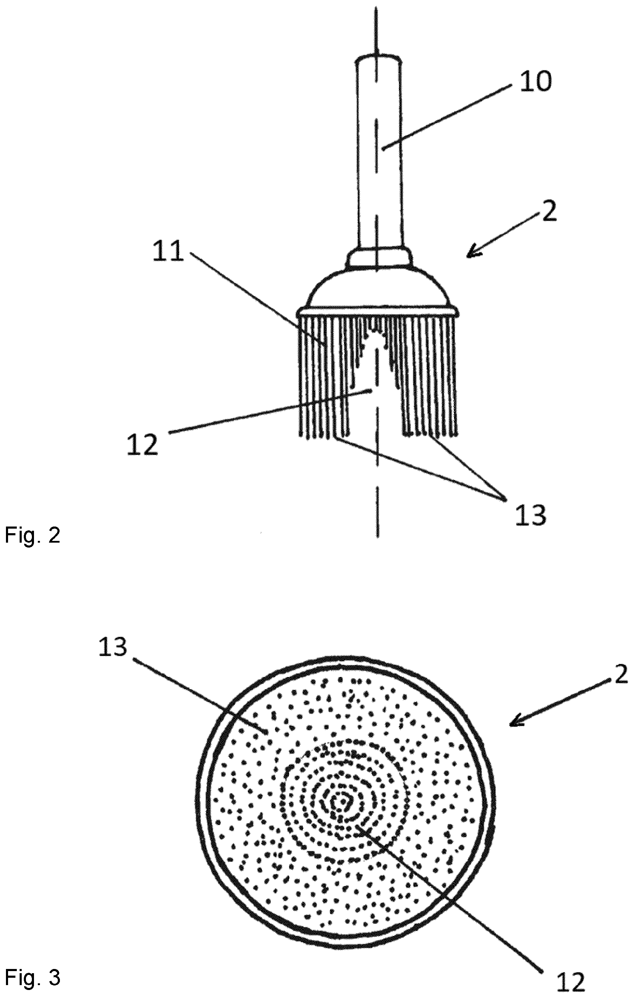

Die Pinselaufsätze 2 (Fig.2 und 3) weisen eine gleichmäßig dichte Haaranordnung 11 aus weichen Naturhaaren aus z.B. Ziegenhaar oder weichen Kunsthaaren auf. Keine Borsten, da diese beim Aufsetzen der Pinselhaube 11 auf die Zecke den eiförmigen weichen Zeckenkörper 14 quetschen oder verletzen könnten. Zudem können sich die kleinen Zeckenbeine 16 nicht im Pinselkranz verfangen. Am freien Ende der Pinselhaare weist die Haaranordnung 11 in der Mitte der Pinselfläche eine kreisförmige Vertiefung 12 in das Pinselinnere hinein auf.

Diese Vertiefung 12 nimmt mit zunehmender Tiefe in kreisrunden Querschnitten vom Durchmesser her so ungleichmäßig ab, dass ihre geometrische dreidimensionale Gesamtform am tiefsten Punkt parabelförmig wie eine Haube der Form eines Zeckenkörpers 14 oder eines Fingerhutes gleicht.

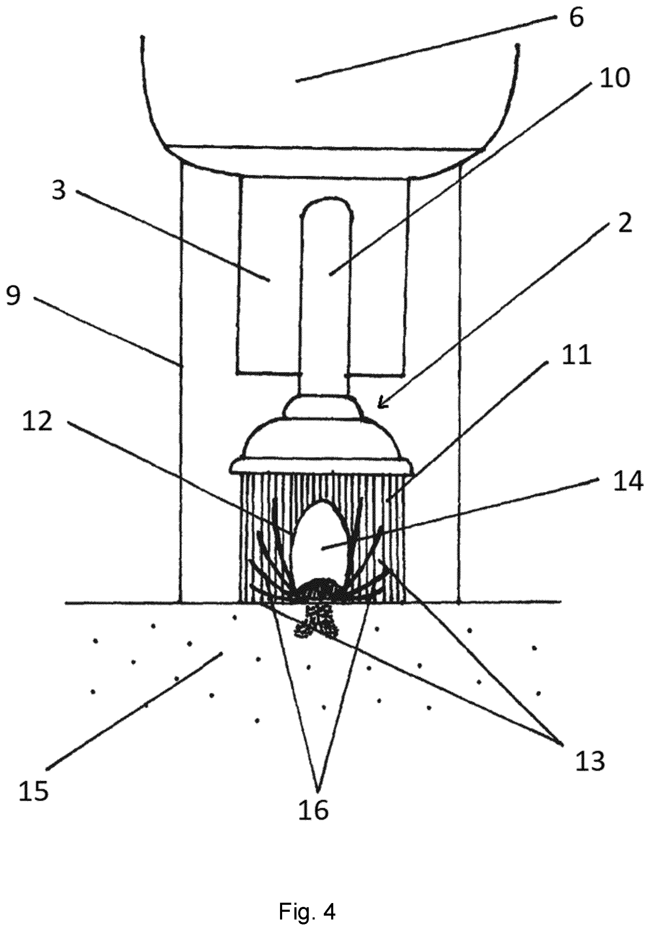

Diese entscheidende Form der Vertiefung 12 hat mit ihrem entstandenen dreidimensionalen Raum im Inneren des Pinsels die Aufgabe, durch das Aufsetzen des Pinselaufsatzes 2 mit dem Gerät (der Vorrichtung 1) über den Zeckenkörper 14 (Fig.4), diesen durch ihre Form mit den weichen Pinselhaaren wie eine Haube zu umschließen ohne ihn zu quetschen oder zu verletzen, sodass sich die Zecke 14 im Inneren des Pinselaufsatzes 2 mit ihren verfangenen Beinen 16 in der beschriebenen Vertiefung 12 / Haube und im Pinselkranz 5 beim Einschalten des Gerätes 1 mit der Rotation mitdrehen kann.

Mit bisherigen existierenden Pinselaufsätzen, wie z.B. aus der Zahnmedizin ist eine erfolgreiche gefahrlose Zeckenentfernung nicht gewehrleistet und mit einem Verletzungs- bzw. Abrissrisiko der Zecke verbunden. Weiter ist nur in Verbindung mit den oben beschriebenen Pinselaufsätzen auch die konstante, gleichmäßige und kontinuierliche Umdrehungszahl der Pinselaufsätze für ein sanftes risikofreies Entfernen der Zecke aus der Haut von Mensch und Tier wichtig.

Ein zu schnelles Drehen oder Vibrieren, wie z.B. bei einer Zahnbürste oder einem Dentaldremel würde das Abrissrisiko des Zeckenkörpers erhöhen. Zudem sollen sich, durch die beschriebene langsame Rotationsgeschwindigkeit die Beine 16 der Zecke 14 in dem Pinselkranz 13 verfangen um den Körper der Zecke 14 dann besser drehen zu können.

### 2. Aufnahmevorrichtung für Pinselaufsätze:

Vorne am Tick-Rotator befindet sich die Aufnahmevorrichtung 3 für die drei variabel fixierbaren Pinselaufsätze 2.

Diese Aufnahmevorrichtung 3 kann z.B. ein kleines Bohrfutter, eine Steckklemme oder ähnliches aus Metall oder Kunststoff sein und ist auf der Antriebswelle fest verbaut. Sie dient dazu, die Pinselaufsätze 2 festzuhalten. Durch einfaches Auf- und Zudrehen kann der Pinselaufsatz 2 bei Bedarf je nach Zeckengröße problemlos gewechselt werden.

Ein Dreh- oder ein Steckmechanismus kommt hier infrage.

### 3. Elektromotor mit Getriebe und Antriebswelle:

Der Elektromotor 4 treibt das Getriebe mit seiner Antriebswelle an. Dieser arbeitet mit 1,5 Volt und erzeugt, reduziert durch das Getriebe, am Ende etwa 60 bis max. 150 Umdrehungen pro Minute.

Die gleichmäßige Umdrehungszahl darf nicht zu hoch sein, da zum einen die Zecke 14 zu schnell gedreht werden kann und zum anderen eine unnötige Reizung der Haut 15 durch Reibungswärme entstehen kann. Auch Tierhaare zum Beispiel bei einem Hund können sich so nicht verfangen.

Das gesamte kleine Getriebe selbst mit Zahnrädern und Achsen kann ebenso aus Metall oder Kunststoff bestehen.

Der Motor und das Getriebe befinden sich im vorderen Teil des Gehäuses. Die Aufnahmevorrichtung 3 befindet sich gut bedienbar außerhalb des Gehäuses.

### 4. Gehäuse mit Batterie und Verkabelung:

Das Gehäuse des Tick-Rotators besteht aus Metall oder Kunststoff und sichert eine stabile Aufnahme des Motors mit seinem Getriebe, der Elektrik und der Energieträger im Inneren. Es hat eine handliche, griffsichere und rutschfeste zylindrische Form.

Im Betrieb mit der Rotation hält man den Tick-Rotator 1 wie eine Pinzette mit Daumen und Zeigefinger einfach über die Zecke.

Am Ende hat er ein kleines Gewinde auf das der Gehäusedeckel 8 mit Ein- und Ausschalter gedreht wird.

Der Energieträger 7 ist eine handelsübliche Batterie oder ein Akku.

### 5. Gehäusedeckel mit Schalter:

Auch der Gehäusedeckel besteht aus dem Material oder Kunststoff wie das Gehäuse und hat ein kleines Gegengewinde um ihn auf das Gehäuse zu schrauben.

Im Deckel befindet sich ein Druckschalter zum Ein- und Ausschalten des Tick-Rotators und der elektrische Pol mit Druckfeder für die Batterie.

### 6. Zentrierhülse:

Die durchsichtige Zentrierhülse 9 aus Kunststoff oder Glas befindet sich abnehmbar an der vorderen Spitze des Tick-Rotators 1. Sie ist zylindrisch vorne am Gehäuse aufgesteckt und hat zwei wesentliche Aufgaben.

Erstens ist sie eine Schutzhülle für den Pinselaufsatz 2, um einen unnötigen Verschleiß oder Verbiegungen des Pinsels zu vermeiden.

Wichtiger ist aber, dass der Benutzer durch den Aufsatz der Hülse 9 auf der Haut 15 um die Zecke 14 herum einen rutschfesten, sicheren und auch genau zentrierten, mit der Zecke 14 in der Mitte des Hülsenkreises, Gebrauch durchführen kann. Durch das genaue Aufsetzen der durchsichtigen Zentrierhülse 9 mit der Zecke 14 in der Mitte sitzt der Tick-Rotator 1 mit seiner Kegelaussparung im Pinsel genau auf der Zecke. Nun kann mit der Rotation begonnen werden.

Die Zentrierhülse 9 hat einen Durchmesser von ca. 10 bis 15mm.

Vorne schließt die Zentrierhülse 9 genau mit dem Ende der Randpinselhaare ab, sodass sowohl Pinsel als auch Hülse 9 gleichzeitig die Haut 15berühren. Außerdem verhindert die Hülse 9, dass die Zecke 14 nach dem Loslassen nicht herunterfällt. Die Hülse 9 ist jedoch für geübte Benutzer nicht zwingend notwendig.

Ebenso kann der Tick-Rotator 1 auch bei der Benutzung an schwer zugänglichen Körperstellen ohne Zentrierhülse 9 angewendet werden.

Zusammenfassend ermöglicht der Tick-Rotator 1 bei Mensch und Tier und gerade bei kleinen Kindern jeden Alters ein angstfreies, unkompliziertes und sanftes Entfernen einer Zecke aus der Haut. Nur ein leichtes "Kitzeln" ist auf der Haut zu spüren.

Auch bei mehrfachen Versuchen ist ein Abriss des Zeckenkörpers noch nicht vorgekommen.

Auch ist eine Version des Tick-Rotators 1 aus Edelstahl denkbar, die beispielsweise zudem mit Netzbetrieb elektrisch arbeitet..

Der Tick-Rotator 1 ist vorteilhaft klein, handlich, transportabel und kann von jedem überall angewendet werden.

Vor der Benutzung ist ein Funktionstest in Bezug auf den Ladezustand der Energieträger 7 notwendig.

Nach der Benutzung sollte der Pinselaufsatz 2 gereinigt, desinfiziert oder bei Notwenigkeit ausgetauscht werden. Ebenso sollte die Wunde bzw. die Einstichsteile nach einer Benutzung mit Desinfektionsspray desinfiziert werden.

Durch die Benutzung des Tick-Rotators 1 kann also viel Leid und Angst vermieden werden. Ein schnelles, unkompliziertes, sicheres und komplettes Entfernen auch der kleinsten Zecke nach einem Stich ist von bedeutender Wichtigkeit in Bezug auf übertragbare Krankheitserreger und risikofrei mit dem Tick-Rotator umsetzbar.

Die Vorrichtung 1 dient also zur sanften Entfernung von Zecken aus der Haut von Mensch und Tier ohne Greif-, Schlingen- oder Hebelmechanismus.

### Bezugszeichenliste

- 1: Vorrichtung/ Tick-Rotator
- 2: Pinselaufsatz
- 3: Aufnahmevorrichtung für Pinselaufsätze
- 4: Elektromotor
- 5: Getriebe
- 6: Gehäuse mit Griff
- 7: Batterie/Akku
- 8: Gehäusedeckel mit Schalter
- 9: Zentrierhülse
- 10: Aufnahmestift
- 11: Haaranordnung
- 12: Vertiefung
- 13: Pinselkranz
- 14: Zecke
- 15: Haut
- 16: Beine der Zecke

## Patentansprüche

1. Vorrichtung (1) zur Entfernung von Zecken (14) aus der Haut (15) eines Menschen oder eines Tieres, aufweisend
- ein Gehäuse mit einem an diesem ausgebildeten Griff,
- einen drehbaren Pinselaufsatz (2), und
- eine Aufnahmevorrichtung (3) zur Aufnahme des Pinselaufsatzes (2),
- wobei der Pinselaufsatz (2) eine Haaranordnung (11) mit Pinselhaaren aufweist, die aus einer Gruppe ausgewählt sind, welche Gruppe Kunsthaare und Naturhaare aufweist, und
- wobei die Haaranordnung (11) des Pinselaufsatzes (2) eine mittig vorgesehene Vertiefung (12) für das Platzieren eines Zeckenkörpers ausbildet,
**dadurch gekennzeichnet,**
**dass** eine durchsichtige Zentrierhülse (9) aus Kunststoff oder Glas abnehmbar am Gehäuse aufgesteckt ist, wobei die Zentrierhülse (9) mit dem freien Ende der Randpinselhaare abschließt.

2. Vorrichtung (1) nach Anspruch 1,
wobei ein elektrischer Antrieb für das Drehen des Pinselaufsatzes (2) vorgesehen ist.

3. Vorrichtung (1) nach Anspruch 1 oder 2,
ferner mit einem Elektromotor (4), der eine Antriebswelle aufweist, und mit einem Getriebe, wobei der Elektromotor (4) gestaltet ist, um das Getriebe mit seiner Antriebswelle anzutreiben.

4. Vorrichtung (1) nach Anspruch 3,
wobei der Elektromotor (4) und das Getriebe so gestaltet sind, dass ausgangsseitig des Getriebes eine Drehzahl von 60 bis 150 Umdrehungen pro Minute erzeugbar ist.

5. Vorrichtung (1) nach einem der Ansprüche 3 und 4,
wobei der Elektromotor (4) und das Getriebe in dem Gehäuse angeordnet sind.

6. Vorrichtung (1) nach einem der vorangehenden Ansprüche,
wobei die Aufnahmevorrichtung (3) außerhalb des Gehäuses angeordnet ist.

7. Vorrichtung (1) nach einem der vorangehenden Ansprüche,
wobei die Aufnahmevorrichtung (3) zur Aufnahme des Pinselaufsatzes (2) eine Aufnahmevorrichtung (3) zur lösbaren Aufnahme des Pinselaufsatzes (2) ist.

8. Vorrichtung (1) nach einem der vorangehenden Ansprüche,
wobei ein Gehäusedeckel vorgesehen ist, der als Schraubdeckel gestaltet ist und auf das Gehäuse schraubbar ist.

9. Vorrichtung (1) nach Anspruch 8,
wobei der Gehäusedeckel mit einem Ein- und Ausschalter versehen ist.

10. Vorrichtung (1) nach einem der vorangehenden Ansprüche,
wobei eine Batterie oder ein Akku als Energieträger (7) vorgesehen und in dem Gehäuse angeordnet ist.

11. Vorrichtung (1) nach einem der vorangehenden Ansprüche, wobei der Pinselaufsatz (2) für seine Fixierung in der Aufnahmevorrichtung (3) rückseitig einen Aufnahmestift (10) aus Metall oder Kunststoff aufweist.

## Claims

1. Device (1) for removing ticks (14) from the skin (15) of a human or animal, having
- a housing with a handle formed thereon,
- a rotatable brush attachment (2), and
- a receiving device (3) for receiving the brush attachment (2),
- wherein the brush attachment (2) has a hair arrangement (11) with brush hairs selected from a group comprising synthetic and natural hairs, and
- wherein the hair arrangement (11) of the brush attachment (2) forms a centrally provided recess (12) in which a tick body is placed,
**characterized**
**in that** a transparent centring sleeve (9) made of plastic or glass is removably attached to the housing, the centring sleeve (9) ending with the free end of the edge brush hairs.

2. Device (1) according to Claim 1,
wherein an electric drive is provided for rotating the brush attachment (2).

3. Device (1) according to Claim 1 or 2,
further having an electric motor (4), which has a drive shaft, and having a transmission, the electric motor (4) being designed to use its drive shaft to drive the transmission.

4. Device (1) according to Claim 3,
wherein the electric motor (4) and the transmission are designed such that a rotational speed of 60 to 150 revolutions per minute can be produced on the output side of the transmission.

5. Device (1) according to either of Claims 3 and 4,
wherein the electric motor (4) and the transmission are arranged in the housing.

6. Device (1) according to any one of the preceding claims,
wherein the receiving device (3) is arranged outside the housing.

7. Device (1) according to any one of the preceding claims,
wherein the receiving device (3) for receiving the brush attachment (2) is a receiving device (3) for releasably receiving the brush attachment (2).

8. Device (1) according to any one of the preceding claims,
wherein a housing cover is provided which is designed as a screw cover and can be screwed onto the housing.

9. Device (1) according to Claim 8,
wherein the housing cover is provided with an on and off switch.

10. Device (1) according to any one of the preceding claims,
wherein a battery or a rechargeable battery is provided as the energy source (7) and is arranged in the housing.

11. Device (1) according to any one of the preceding claims, wherein the rear side of the brush attachment (2) has a receiving pin (10) made of metal or plastic for fixing it in the receiving device (3).

## Revendications

1. Dispositif (1) pour enlever des tiques (14) de la peau (15) d'un être humain ou d'un animal, présentant
- un boîtier avec une poignée réalisée sur celui-ci,
- un embout de pinceau rotatif (2), et
- un dispositif de réception (3) pour recevoir l'embout de pinceau (2),
- l'embout de pinceau (2) présentant un agencements de poils (11) avec des poils de pinceau choisis dans un groupe, lequel groupe présentant des poils synthétiques et des poils naturels, et
- l'agencement de poils (11) de l'embout de pinceau (2) réalisant une cavité (12) prévue au centre pour le placement d'un corps de tique,
**caractérisé**
**en ce qu'**une douille de centrage (9) transparente en matière plastique ou en verre est enfichée de manière amovible sur le boîtier, la douille de centrage (9) se terminant par l'extrémité libre des poils de pinceau de bordure.

2. Dispositif (1) selon la revendication 1,
un entraînement électrique étant prévu pour la rotation de l'embout de pinceau (2).

3. Dispositif (1) selon la revendication 1 ou 2,
avec en outre un moteur électrique (4) qui présente un arbre d'entraînement, et une transmission, le moteur électrique (4) étant conçu pour entraîner la transmission avec son arbre d'entraînement.

4. Dispositif (1) selon la revendication 3,
le moteur électrique (4) et la transmission étant conçus de telle sorte qu'une vitesse de rotation de 60 à 150 tours par minute puisse être générée côté sortie de la transmission.

5. Dispositif (1) selon l'une quelconque des revendications 3 et 4,
le moteur électrique (4) et la transmission étant agencés dans le boîtier.

6. Dispositif (1) selon l'une quelconque des revendications précédentes,
le dispositif de réception (3) étant agencé à l'extérieur du boîtier.

7. Dispositif (1) selon l'une quelconque des revendications précédentes,
le dispositif de réception (3) destiné à recevoir l'embout de pinceau (2) étant un dispositif de réception (3) destiné à recevoir de manière amovible l'embout de pinceau (2).

8. Dispositif (1) selon l'une quelconque des revendications précédentes,
un couvercle de boîtier étant prévu, lequel est conçu sous forme de couvercle à visser et peut être vissé sur le boîtier.

9. Dispositif (1) selon la revendication 8,
le couvercle du boîtier étant muni d'un interrupteur marche/arrêt.

10. Dispositif (1) selon l'une quelconque des revendications précédentes,
une batterie ou un accumulateur étant prévu en tant que support d'énergie (7) et étant agencé dans le boîtier.

11. Dispositif (1) selon l'une quelconque des revendications précédentes, l'embout de pinceau (2) présentant du côté arrière, pour sa fixation dans le dispositif de réception (3), une tige de réception (10) en métal ou en matière plastique.
